# EUROPEAN PATENT APPLICATION

(11) **EP 3 248 632 A1**
(43) Date of publication of application: **29.11.2017**
(21) Application number: 17161488.6
(22) Date of filing: 17.03.2017
(51) Int. Cl.: A61M 5/14, A61M 5/142, A61M 39/24

(54) **DEVICE FOR STORAGE AND RECONSTITUTION OF A SUBSTANCE FOR ADMINISTRATION TO A SUBJECT**

(71) Applicant: Citros VOF, 9831 Deurle (BE)
(72) Inventor: Moerman, Piet, 9831 Deurle (BE)
(74) Representative: De Clercq & Partners

(57) **Abstract**

Provided is a device (500) for storage and reconstitution of one or more discrete doses of a substance in a pressurised carrier liquid, comprising a first body (100) comprising: at least one discrete storage reservoir (110) configured for storage of the dose of substance, wherein the reservoir is in fluid connection with an inlet channel (112) and an outlet channel (114), wherein the inlet channel (112) is disposed with an inlet pressure-activated valve (140), configured to open responsive to a force of the pressurised carrier liquid, whereby the inlet channel (112) is connected at an inlet end to a common inlet conduit (130), and whereby the outlet channel (114) is connected at an outlet end to a common outlet conduit (132) configured for mixing the substance in the carrier liquid.

## Description

### Field of the invention

The present invention is in a field of substance delivery to a subject. In particular in the field of autonomous systems for storage, selection and reconstitution (e.g. dilution, suspension) and delivery of a dose of a substance to a subject.

### Background to the invention

Glucagon is used to counteract the actions of insulin in diabetic patients. Insulin will lower blood glucose levels while glucagon elevates glucose levels. In cases of imminent hypoglycemia due to a relative overdose of insulin or too little carbohydrate intake, the patient will be given a dose of glucagon. This glucagon is stored as a powder and is reconstituted by adding a diluent just prior to the injection. Glucagon is not stable in a liquid form and loses its functionality within hours.

Newer therapy forms where insulin is given by a pump system are best combined with a glucagon infusion system that can readily interfere in an automated manner when the glucose concentration is predicted to reach low levels. One way of doing this is to combine an insulin pump with a glucagon pump.

The latest closed loop systems for regulating glucose levels prefer the use of a glucose level lowering drug (Insulin) and a glucose level augmenting drug (Glucagon). While the insulin keeps the patient's glucose level from going to high, it is the glucagon that protects the patient from dangerous low glucose levels. Hypoglycemia (low glucose levels) can have severe consequences such as: loss of concentration, blurred vision, confusion and coma.

The two hormones are administered as needed on a semi-continuous basis; similar to the functioning of a healthy pancreas. In patients with diabetes, a closed loop pump allows the two hormones to be infused within seconds. Such a closed loop system closes the loop between the continuous glucose monitor and the delivery of those hormones in the appropriate doses. The closed loop system will do this by using two pumps attached to 2 subcutaneous infusion sets. These pumps are in a continuous standby mode, always ready to intervene for correcting the glucose level. That requires a quantity of insulin and glucagon to be readily available in the pump reservoirs. While the liquid insulin reservoir is relatively stable for up to a week, the liquid glucagon is not.

When preserved in the hydrated state for too long, glucagon tends to form fibrils over time. These fibrils or protein clumps are no longer active. Therefore, glucagon powder needs to be reconstituted immediately prior to its injection. Up to now, chemical or recombinant approaches have failed to form a stable liquid glucagon formulation.

Currently various substances are difficult to formulate in a stable liquid form , for instance certain proteins, protein fragments, viruses and the like, are often instable in a liquid state. Some proteins need serious formulation efforts to be stable in a liquid solution. This is a time and money consuming effort that seriously lengthens the drug development time. Storing the drug in a dry (lyophilized) powder format is the easiest, fastest and the lowest cost format.

An aim of the invention is to provide a device for improving the delivery as a liquid in an automated form while guaranteeing the stability of the substance such as glucagon.

### Summary of some aspects

Provided herein is a device (500) for storage and reconstitution of one or more discrete doses of a substance in a pressurised carrier liquid, comprising a first body (100) comprising:
- at least one discrete storage reservoir (110) configured for storage of the dose of substance, wherein the reservoir is in fluid connection with an inlet channel (112) and an outlet channel (114),
- wherein the inlet channel (112) is disposed with an inlet pressure-activated valve (140), configured to open responsive to a force of the pressurised carrier liquid,
- whereby the inlet channel (112) is connected at an inlet end to a common inlet conduit (130), and
- whereby the outlet channel (114) is connected at an outlet end to a common outlet conduit (132) configured for mixing the substance in the carrier liquid.

The common outlet conduit (132) may be provided with a region (134) dimensioned to facilitate turbulent flow over laminar flow, optionally having for a diameter D in metres, and a Reynolds number ReD >4000.

The outlet channel (114) may be disposed with an outlet pressure-activated one-way valve (160), configured to open responsive to force of the pressurised liquid carrier exiting the storage reservoir (110).

The outlet pressure-activated one-way valve (160) may comprise:
- a diaphragm (164) provided in a wall (163) of the outlet channel (114), and
- a seating member (162) disposed in a void space of the outlet channel (114)
wherein the diaphragm (164) forms a fluidly sealing contact with the seating member (164) to seal the outlet channel (114),
wherein the diaphragm (164) is provided across a recess (166) in the wall (163) of outlet channel (160) and is with an aperture (168) at an outlet side configured for the passage of back-flowing liquid into the recess (166) wherein back-flowing liquid fills the recess (166) via the aperture (168) expanding the diaphragm (164) and sealing it against the seating member (162) to prevent entry of the back-flowing liquid into the storage reservoir (110).

The device may have a plurality of reservoirs (110), wherein the reservoirs (110) and connected inlet (112) and outlet (114) channels are arranged in an arc or circle, the inlet channels (112) disposed towards a periphery, the outlet channels (114) arranged towards a centre, and the reservoirs (110) disposed between the inlet and outlet channels.

Downstream of the inlet valve (140) a flow restrictor (400) may be provided increasing the flow resistance (400) causing sufficient resistance to create sufficient pressure in the liquid flow to open the inlet valve (140)

Each of the inlet pressure-activated valves (140) may be selectively lockable in a closed position.

The lockable inlet pressure-activated valve (140) may comprise:
- a diaphragm (144) provided in a wall (142) of the inlet channel (112),
- a seating member (146) disposed in a void space of the inlet channel (112), wherein the diaphragm (144) forms a fluidly sealing contact with the seating member (146) to seal the inlet channel (112),
- a moveable clamping element (150) configured to apply a locking force to the diaphragm (144) against the seating member (146) from an exterior of the wall (142) of the inlet channel (112) to lock it in a closed position.

The device (500) may further comprise a selector unit (200) configured for dismountable attachment to the first body (100), and to lock a selected subset of the inlet pressure activated-valves (140) in a closed position, thereby directing the pressurised carrier liquid through one or more inlet pressure-activated valves not in the subset.

The selector unit (200) may be configured to change the composition of the subset between consecutive pulses of the pressurised carrier liquid.

The selector unit (200) may comprise:
- a plurality of electro-mechanical actuators (292) in a normally-locked state each configured to apply a locking force to a clamping element (150), and
- a controller configured to activate one electro-mechanical actuator at a time, thereby unlocking one of the clamping elements at the time (150).

The selector unit (200) may comprise a pressure-activated spring-loaded releasable ratchet mechanism configured to release a moveable pawl (216, 218) one ratchet position responsive to a pressure pulse of the carrier liquid, wherein the position of the pawl (216, 218) is in alignment relative to a position of the one or more clamping elements (150) in a non-locked position.

The selector unit (200) may comprise:
- an upper disc (260) and a lower disc (210) in mutual revolute relation around their respective central axes, the lower disc (210) provided for rotation relative to the first body (100), and the upper disc provided to be in fixed revolute relation with the first body (100),
- a spring (220) disposed between the lower disc (210) and the upper disc (260) to supply torque to rotate the lower disc (210) between ratchet positions,
- a first releasable ratchet comprising a first pawl (216) disposed on a compliant region (214) of the lower disc (210) and a first gear (262) disposed on the upper disc (260),
- a second releasable ratchet comprising a second pawl (21) disposed compliant region (214) of the lower disc (210) and a second gear (180) disposed on the first body (100),
- the first gear teeth (264', 264", 264"') and second gear teeth (182', 182", 182"') are in alignment relative to a positions of the clamping elements,
- wherein the lower disc (210) is configured to apply a locking force to a subset of clamping elements (150),
- wherein the compliant region (214) provided with the first pawl (216) and second pawl (218) is moveable between the first and second gears responsive to movement of the clamping element (150) under force of the pressurised carrier liquid, thereby releasing and moving the ratchet one ratchet position responsive to a pressure pulse.

Also provided is a selector unit (200) as defined herein.

Also provided is a liquid administration device (300) for bodily subcutaneous administration of a first liquid (310) and a second liquid (314), comprising a first fluid conduit (308) for conveying the first liquid (310), and a second fluid conduit (312) for conveying the second liquid (314), the liquid administration device (300) further comprising:
- a cannula (306) having a single lumen in fluid connection with the first fluid conduit (308) and the second fluid conduit (312) arranged to effect mixing of the first (310) and second (314) liquids prior to conveyance through the cannula (306), or
- a single cannula (306) having a first lumen (306a) in fluid connection with the first fluid conduit (308), and a second lumen (306b) in fluid connection with the second fluid conduit (312), the first lumen (306a) outlet and second lumen (306b) outlet arranged to effect mixing of the first (310) and second (314) liquids within the body, optionally wherein the first cannula (306a) and a second cannula (306b) are disposed adjacently or coaxially,
   or
- a first cannula (306a) in fluid connection with the first fluid conduit (308), and a second cannula (306b) in fluid connection with the second fluid conduit (312), both first cannula (306a) and second cannula (306b) outlets arranged in close proximity to effect mixing of the first (310) and second (314) liquids within the body,
optionally wherein the liquid administration device (300) is configured for dismountable attachment to the device (500) according to any of claims 1 to 13, and for fluid connection of the first (308) or second (312) fluid conduit to the common outlet conduit (132).

### Figure Legends

**FIG. 1** depicts an example of a first body disposed with a plurality of storage reservoirs for containing doses of a substance.
**FIG. 2** depicts an example of an inlet pressure-activated valve (closed position).
**FIG. 3** depicts an example of an inlet pressure-activated valve (open position).
**FIG. 4** depicts an example of a lockable inlet pressure-activated valve (locked closed position).
**FIG. 5** depicts an example of a lockable inlet pressure-activated valve (unlocked closed position).
**FIG. 6** depicts an example of an outlet pressure-activated one-way valve (open position, forward flow).
**FIG. 7** depicts an example of an outlet pressure-activated one-way valve (closed position, backward flow).
**FIG. 8A** is a schematic illustration of a selector unit undocked from a first body.
**FIG. 8B** is a schematic illustration of a selector unit attached to a first body.
**FIG. 9** is a schematic illustration of a first body of FIG. 1 disposed with a second gear component of a second ratchet.
**FIG. 10** is a schematic illustration of a lower disc of the selector unit (upper surface).
**FIG. 11A** is a schematic illustration of an upper disc of the selector unit (upper surface).

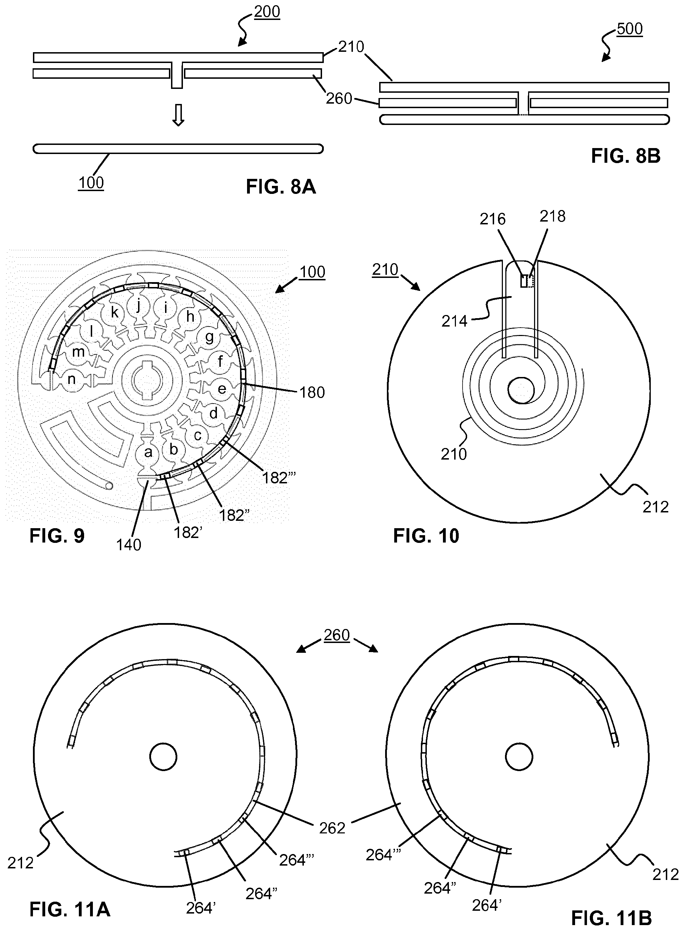

**FIG. 11** **B** is a schematic illustration of an upper disc of the selector unit (lower surface).

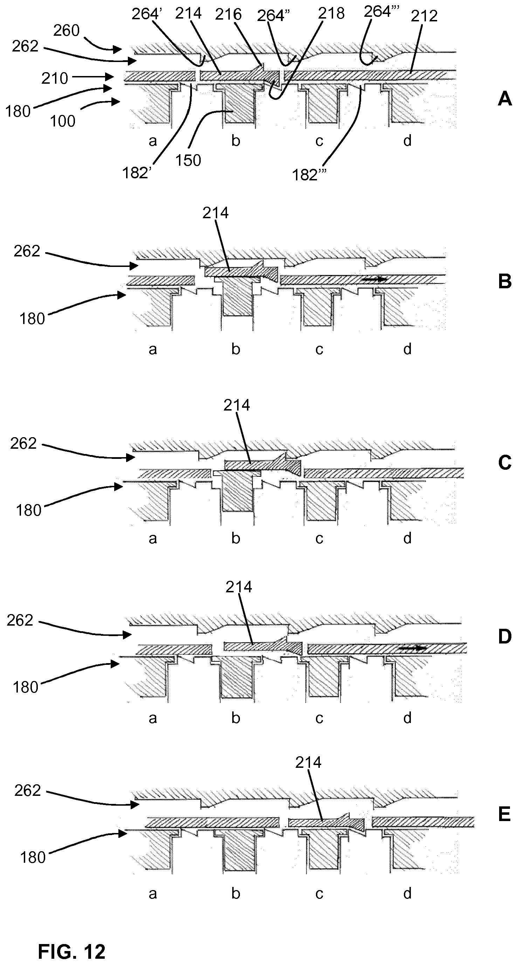

**FIGs. 12** **A** to **E** are schematic illustrations a double ratchet mechanism, and advancement of a pawl by one ratchet position responsive to a pressure pulse.

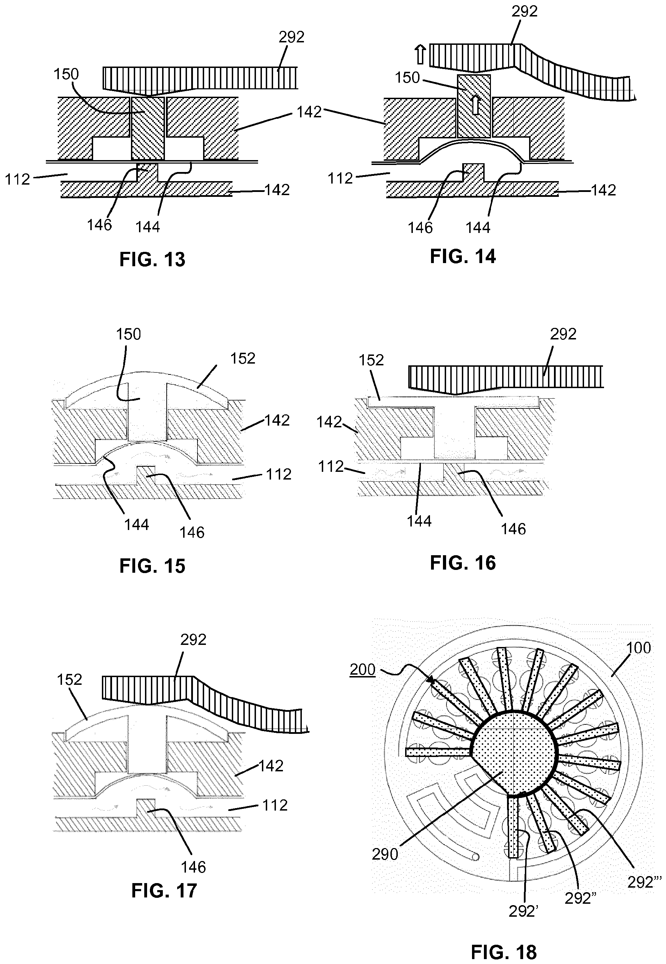

**FIG. 13** is a schematic illustration of a clamping element maintained in a locked position by force of a piezo-electric arm.
**FIG. 14** is a schematic illustration of a clamping element of FIG. 13 in an unlocked position where the piezo-electric arm is raised, and the clamping element is displaced upwards due to pressure of the liquid carrier on the pressure activated inlet valve.
**FIG. 15** is a schematic illustration of a clamping element disposed with a compliant dome-shaped flange.
**FIG. 16** is a schematic illustration of the clamping element of FIG. 15 maintained in a locked position by the passive force of a piezo-electric arm.
**FIG. 17** is a schematic illustration of the clamping element of FIG. 15 in an unlocked position where the piezo-electric arm is actively raised, and the clamping element is displaced upwards due to pressure of the liquid carrier on the pressure activated inlet valve.
**FIG. 18** is a schematic illustration of a plurality of piezo-electric arms radiating from a central chassis.

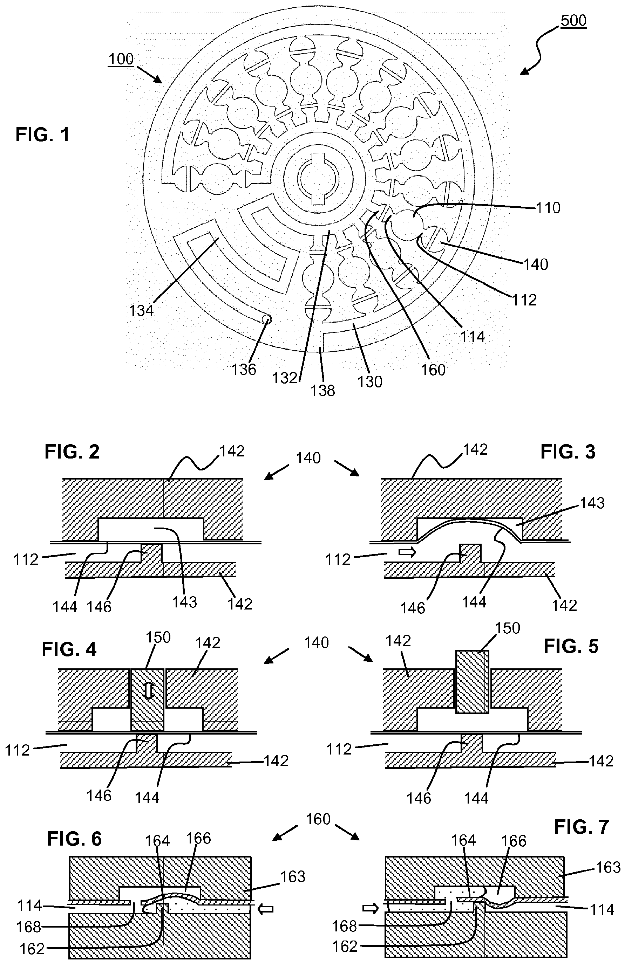

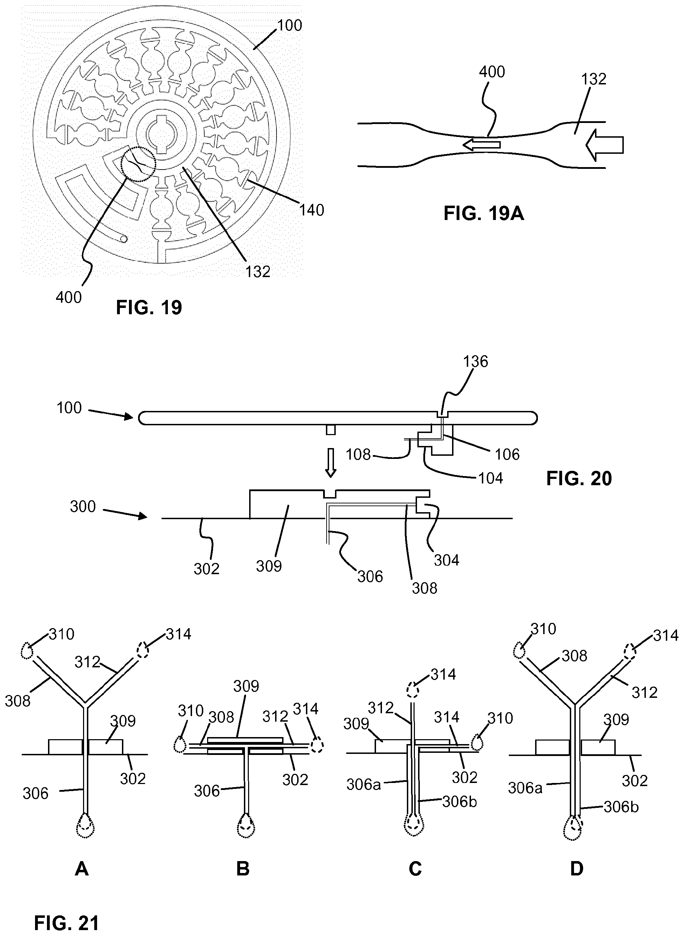

**FIG. 19** is a view of the first body of FIG. 1 provided with a flow restrictor; FIG. 19A is a detail of the flow restrictor.
**FIG. 20** is a view of the first body and an administration device as separated parts.
**FIG. 21A** to **D** illustrate different examples of a dual substance administration device.

### Detailed description of invention

Before the present device is described, it is to be understood that this invention is not limited to particular devices described, since such devices and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, *e.g*., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. It is to be understood that other embodiments may be utilised and structural or logical changes may be made without departing from the scope of the present invention. The following detailed description, therefore, is not to be taken in a limiting sense, and the scope of the present invention is defined by the appended claims.

Described herein is a device (500) for storage and reconstitution (*e.g*. by dilution or suspension) of one or more discrete doses of a substance in a liquid carrier.

The device (500), as shown, for instance, in **FIG. 1** comprises:
- at least one discrete storage reservoir (110) configured for storage of the dose of the substance, wherein the reservoir (110) is in fluid connection with an inlet channel (112) and an outlet channel (114),
- wherein the inlet channel (112) is disposed with an inlet pressure-activated valve (140), configured to open responsive to force of the pressurised liquid carrier,
- wherein the inlet channel (112) is connected at an inlet end to a common inlet conduit (130), and
- wherein the outlet channel (14) is connected at an outlet end to a common outlet conduit (132)

The device may comprise two or more discrete storage reservoirs (110), accordingly, the device (500) comprises:
- at least two discrete storage reservoir (110) each configured for storage of the dose of the substance, wherein each reservoir (110) is in fluid connection with an inlet channel (112) and an outlet channel (114),
- wherein each inlet channel (112) is disposed with an inlet pressure-activated valve (140), configured to open responsive to force of the pressurised liquid carrier,
- whereby each inlet channel (112) is connected at an inlet end to a common inlet conduit (130), and
- whereby each outlet channel (14) is connected at an outlet end to a common outlet conduit (132)

The at least one or two discrete storage reservoir (110), inlet channel (112) and an outlet channel (14), inlet pressure-activated valve (22, 24), common inlet conduit (30), and common outlet conduit (32) may be disposed on or within a first body (100). The first body (100) has an inlet side closest to an inlet port (138) and an outlet side closest to an outlet port (136).

Where there is a plurality of reservoirs (110), they, the connected inlet (112) and outlet (114) channels may be arranged in an arc or circle, the inlet channels (112) disposed towards a periphery, the outlet channels (114) arranged towards a centre, and the reservoirs (110) disposed between the inlet and outlet channels.

The substance is typically a solid, for instance, a powdered, a granulated substance, a lyophilised mass or a gel. The substance may be any that is unstable in liquid form and stable in solid form such as glucagon. The substance may be a protein, nucleic acid, biological material such as a virus. The substance may be dissolvable or suspendable in the liquid carrier. The liquid carrier may be any biologically-compatible liquid, for instance, buffered saline solution. Where the substance is glucagon, the liquid carrier may be made from 12 mg/ml glycerin, purified water and hydrochloric acid.

The first body (100) comprises at least one or two discrete storage reservoirs (110) each configured for storage of the dose of a substance. Preferably the storage reservoirs (110) are each configured for the dry storage of the dose of a substance. It further comprises at least one or two inlet channels (112) and outlet channels (114), each in fluid connection with a storage reservoir. It further comprises at least one or two inlet pressure-activated valves each in connection with inlet channel (112), each configured to open responsive to force of the pressurised liquid carrier. It further comprises a common inlet conduit (130), and a common outlet conduit (132). The liquid conducting features are preferably formed within the body.

The body may have the form of a flattened geometric shape, for instance, rectangular (oblong, square), preferably a disc shape.

The body may be manufactured from two or more pieces (e.g. halves) that are assembled, optionally with a layer (elastomer layer) between forming the valve diaphragms (see below). The structure of the at least one or two discrete storage reservoirs (110), inlet channels (112) and outlet channels (14), inlet pressure-activated valves (22, 24), common inlet conduits (30), and common outlet conduits (32) may be defined by the two or more pieces. These two or more pieces may be made from polymeric material (e.g. polystyrene, poly-metyl-mete-acrylate PMMA, poly-etylene-tetra-phtalate-glycol PETG, polypropylene PP, or others). The two or more pieces may be embossed, imprinted, milled or injection moulded. An elastomeric layer may be used to make the valves diaphragms (see below).

The first body (100) may be configured for dismountable attachment to a bodily administration device configured to administer the dose subcutaneously, intravenously, intramuscularly, orally, by inhalation, or by any other route. The first body (100) may be configured for dismountable attachment to a selector unit (200), configured to control, namely restrict flow of the liquid carrier though a subset of the inlet pressure-activated valves. The first body (100) may be configured for dismountable attachable to a pump.

The storage reservoir (110) is configured to maintain the dose in a dry condition. It is sealed from the atmosphere. The inlet pressure-activated valve (140) seals the storage reservoir (110) from the atmosphere. The outlet pressure-activated valve (160) further seals the storage reservoir (110) from the atmosphere. The number of storage reservoirs (110) is at least one, at least 2, for instance, at least 5, at least 10, preferably at least 5-25. In the example of **FIG. 1****,** the first body (100) is provided with 14 discrete storage reservoirs (110). Each and every storage reservoir (110) may be provided with a dose of substance. One of the storage reservoirs may act as a priming conduit e.g. to prime the common inlet conduit (130) and the common outlet conduit (132) with carrier liquid.

Each inlet channel (112) is disposed with an inlet pressure-activated valve (140), configured to open responsive to force of the pressurised liquid carrier. The inlet pressure-activated valve (140) is biased in a closed and fluidly-sealing configuration, *i.e.* it is a normally-closed valve. The pressured liquid carrier entering the inlet channel (112) applies an opening force to the inlet pressure-activated valve (140), thereby exposing the content of the storage reservoir (110) to the pressured liquid carrier.

The inlet pressure-activated valve (140) may further be lockable. When locked, the valve (140) is locked in the closed configuration, and the force of pressured liquid carrier entering the inlet channel (112) is insufficient to open the inlet pressure-activated valve (140), thereby preventing exposure of the content of the storage reservoir (110) to the pressured liquid carrier. When unlocked, the valve (140) is configured to open responsive to force of the pressurised liquid carrier. By providing a lockable inlet pressure-activated valve (140) a subset of inlet pressure-activated valves (140) may be selected (locked) to prevent flow of pressured carrier liquid into the selected storage reservoirs (110), and permit flow into one or more storage reservoirs (110) connected to unlocked inlet pressure-activated valves (140).

The inlet pressure-activated valve (140) may comprise a combination of a diaphragm (144) provided in a wall (142) of the inlet channel (112) and a seating member (146) disposed in a void space of the inlet channel (112), wherein the diaphragm (144) forms a fluidly sealing contact with the seating member (146), as shown, for instance, in **FIG. 2****.** Upon on the application of sufficient force by the pressured liquid carrier, the diaphragm (144) is separated from the seating member (146), and pressured liquid carrier may flow in a gap formed between the diaphragm (144) and seating member (146) and towards the storage reservoir (110), as shown, for instance, in **FIG. 3****.** The diaphragm (114) is shown lifted into a recess (143). The diaphragm (144) may be an expandable membrane e.g. an elastomer. The seating member (146) is typically an inward projection from the wall of the inlet channel (112).

The inlet pressure-activated valve (140) may be rendered lockable using a clamping element (150) - typically a moveable rigid body configured to contact and apply force to the diaphragm (144) from an exterior of the wall (142) of the inlet channel (112). External force may be applied to the clamping element (150) using a selector unit (200) having mechanical and/or electromechanical components configured to selectively apply an external clamping force to one or more of the clamping elements (150), thereby locking in a closed position an inlet pressure-activated valve (140). The clamping element (150) may be provided as part of the first body (100) or as part of a selector unit (200), or as an independent part.

Each outlet channel (114) may be disposed with an outlet pressure-activated valve (160), configured to open responsive to force of the pressurised liquid carrier directed from the storage reservoir (110). The outlet pressure-activated valve (160) is biased in a closed and fluidly-sealing configuration, *i.e.* it is a normally-closed valve. The pressured liquid carrier exiting the storage reservoir (110) enters the outlet channel (114) applies a force to the outlet pressure-activated valve (160) to open it, thereby allowing the liquid to enter the common outlet conduit (132).

The outlet pressure-activated valve (160) may comprise a combination of a diaphragm (164) provided in a wall (163) of the outlet channel (114) and a seating member (162) disposed in a void space of the outlet channel (114), wherein the diaphragm (164) forms a fluidly sealing contact with the seating member (162). Upon on the application of sufficient force by the pressured liquid carrier, the diaphragm (164) is separated from the seating member (162), and pressured liquid carrier may flow in a gap formed between the diaphragm (164) and seating member (162) and towards the common outlet conduit (132). The diaphragm (164) may be an expandable membrane e.g. an elastomer.

The outlet pressure-activated valve (160) may be a one-way valve configured to permit liquid flow towards the outlet port (136) and preventing flow towards the inlet port (138). The one-way outlet pressure-activated valve (160) may comprise a combination of a diaphragm (164) provided in a wall (163) of the outlet channel (114) and a seating member (162) disposed in a void space of the outlet channel (114) wherein the diaphragm forms a fluidly sealing contact with the seating member (164), as described above. The diaphragm (164) is fluidly sealed over a recess (166) in the wall (163) of outlet channel (160) as shown, for instance, in **FIG. 6****.** The diaphragm (164) is provided with an aperture (168) at an outlet side configured for the passage of back-flowing liquid into the recess (166). When a back-flowing liquid enters the aperture (168), as shown, for instance, in **FIG. 7****,** it fills the recess (166) and applies additional force to the diaphragm (164), expanding it, thereby sealing against the seating member (162) to prevent entry of the back-flowing liquid into the storage reservoir (110).

The common outlet conduit (132) is in fluid connection with each of the outlet channels (114). It directs the liquid carrier containing the substance towards an outlet port (136). The outlet port (136) is configured for dismountable connection to an administration device for administration of the substance-liquid carrier to the body.

The common outlet conduit (132) may be configured to effect mixing of the substance in the carrier liquid. According to one aspect, the common outlet conduit (132) is provided with a region (134) - at least a part of a length of the common outlet conduit (132) - having a transverse cross-sectional profile (e.g. a diameter size) conducive to turbulent flow. One or more passive mixing elements (e.g. one or more protrusions) may be provided in the region to enhance the formation of liquid turbulence. According to one aspect, the region is dimensioned to facilitate turbulent flow over laminar flow. According to one aspect, the sub-region is configured such that, for a flow in the region having a diameter D (in metres), the Reynolds number Re_{D} >4000.

The common inlet conduit (130) is in fluid connection with each of the inlet channels (112). It directs the pressurised carrier liquid towards each of the inlet channels (112). An inlet end of the common inlet conduit (130) is in fluid connection with an inlet port (138). The inlet port (138) is configured for dismountable connection to a pump device for providing the pressurised liquid carrier.

A flow restrictor (400) may be provided configured to increase flow resistance causing a pressure increase at the inlet pressure-activated valve (140). There may be one or more flow restrictors (400). The flow restrictor is configured to allow the pressurised carrier liquid to apply sufficient force to the inlet pressure-activated valve (140) to maintain it in an open position. The flow restrictor (400) may be provided within the first body (100). The flow restrictor (400) may be provided downstream (outlet side) of the inlet pressure-activated valve (140). The flow restrictor (400) may be provided, for instance, in an inlet channel (112) downstream of the inlet pressure-activated valve (140), in the outlet channel (114), and/or in the common outlet conduit (132). The flow restrictor (400) may comprise a zone in a channel or conduit having narrower diameter, and /or a change in surface characteristics, and /or a change in the channel bending compared with the outside the zone. Shown in **FIG. 19** and in detail in **FIG. 19A** is exemplary flow restrictor (400) that is a narrowed zone of the common outlet conduit (132). The flow restrictor (400) may comprise the outlet pressure-activated valve (160); a flow resistance of the outlet pressure-activated valve (160) might suffice to restrict flow. The flow restrictor (400) may be provided in a connection between the outlet port (136) and an administration device (see later below), for instance, in a tube or needle. The flow restrictor (400) may be provided in an administration device (see later below), for instance, in a conduit, cannula or needle. It is appreciated that, depending on the flow per second, the distention of the receiving tissue, or blood pressure in which the carrier liquid is delivered may cause sufficient resistance to act as a flow restrictor (400).

For storage and transportation purposes, the first body (100) may be provided in a sealed foil pouch.

The selector unit (200) is configured to control - namely restrict flow of the liquid carrier though a subset of - the inlet pressure-activated valves. As mentioned previously, the inlet pressure-activated valves (140) may be lockable in a closed configuration, the selector unit (200) provides a clamping force to the subset of the inlet pressure-activated valves (140).

The selector unit (200) is preferably configured to select a subset of inlet pressure-activated valves (140) for locking closed responsive to a pressure pulse of the liquid carrier. For the duration of the pulse of pressure, the carrier liquid passes through the unlocked inlet pressure-activated valves but not through the locked inlet pressure-activated valves. Between consecutive pulses, the selector unit (200) is configured to change the composition of the subset. Typically, the previously unlocked inlet pressure-activated valve (140) becomes locked and a previously next in order, locked inlet pressure-activated valve (140) connected to a storage reservoir containing a dose of substance becomes unlocked. The selector unit (200) is configured such that each pressure-activated valve is unlocked only once, optionally until all the reservoirs have been depleted. It is preferred that the number of inlet pressure-activated valves in the subset is the total number of valves minus 1. It is preferred that unlocked inlet is changed sequentially.

In a preferred aspect, the selector unit (200) in response to a pressure pulse of the carrier liquid, is configured to
- lock all but one of the inlet pressure-activated valves closed,
- change the subset, such that each pressure-activated valve provided is unlocked only once, optionally until all the reservoirs have been depleted

The selector unit (200) is configured for dismountable attachment to the first body (100), as shown for instance in **FIG. 8A** (dismounted) **and** **FIG. 8B** (mounted). Dismountability may be realised by any means, for instance, using a quick-release clip, a screw connection, a bolt connection and the like.

According to one aspect the selector unit (200) comprises a pressure-activated spring-loaded releasable ratchet mechanism configured to release a pawl (216, 218) one ratchet position responsive to a pressure pulse of the carrier liquid, wherein the position of the pawl (216, 218) is in alignment relative to a position of the one or more clamping elements (150) in a non-locked position.

The selector unit (200) may comprise a pair of discs, an upper disc (260) and a lower disc (210) in mutual revolute relation around their respective central axes as shown, for instance, in **FIG. 8B****.** The lower disc (210) provided for rotation relative to the first body (100), and the upper disc provided to be in fixed revolute relation with the first body (100).

The lower disc (210), as shown for instance, in **FIG. 10** is formed from an essentially rigid and planar body (212) and is configured to contact the subset of clamping elements (150), providing a clamping force to retain the subset of corresponding inlet pressure-activated valve (140) in a locked state. The lower disc (210) is provided with a compliant region (214) for co-operating contact with one or more clamping elements (150), biased in a plane of the lower disc (210), and which compliant region (214) is moveable above the plane of the lower disc (210) responsive to movement of the clamping element under force of the pressurised carrier liquid. The compliant region (214) may have a radially fixed position. The compliant region (214) does not apply a clamping force, hence, when it is aligned with one or more clamping elements (150), pressurised carrier liquid is able to open the inlet pressure-activated valve (140) and flow into the corresponding storage reservoir (110). Rotation of the lower disc (210) allows changeable selection of one or more clamping elements (150) and hence storage reservoir (110) for dose delivery.

The upper disc (160), as shown for instance, in **FIGs. 11A** and **11** **B** is configured to be in fixed revolute alignment with the first body (100), when the selector (200) is attached thereto. The lower disc (210) rotates at discrete revolute angles relative to the upper disc (260), wherein the rotation to each discrete angle is actuated by the pressure pulse.

A first releasable ratchet may be disposed between the lower disc (210) and the upper disc (260) as shown in the sequence **FIGs. 12** **A** to **E.** The first releasable ratchet allows a one way rotation of the lower disc (210) relative upper disc (260), and to wind up a spring (220) (e.g. clock spring) disposed between the lower disc (210) and the upper disc (260) which supplies torque to move the lower disc (210) between the discrete revolute angles. In order to rotate under the force of the spring (220), the first releasable ratchet is released one ratchet position, which release is actuated by the pressure pulse. A first pawl (216) of the first releasable ratchet is provided on the compliant region (214) of the lower disc (210), in fixed relation thereto. Specifically it is provided on an upper surface of the compliant region (214). A first gear (262) of the ratchet is disposed of an adjacent surface of the upper disc (260). Specifically it is provided on a lower surface of the upper disc (260). The first gear teeth (264', 264", 264"') are preferably positioned within arc regions of the upper disc corresponding to arc regions between adjacent inlet pressure-activated valves (140) of the first body (100). The first gear teeth (264', 264", 264"') are arranged in an arc or circle.

A second releasable ratchet disposed between the lower disc (210) and the first body (100) allows a one way rotation of the lower disc (210) relative to first body (100). The direction of rotation is the same as that between the lower disc (210) and the upper disc (260). A second pawl (218) of the second releasable ratchet is provided on the compliant region (214) of the lower disc (210), in fixed relation thereto. Specifically it is provided on a lower surface of the lower disc (210). A second gear (180) of the ratchet is disposed of an adjacent surface of the first body (100). Specifically it is provided on an upper surface of the first body (100). The second gear teeth (182', 182", 182"') are preferably positioned within arc regions between adjacent inlet pressure-activated valves (140) of the first body (100). The second gear teeth (182', 182", 182"') are arranged in an arc or circle.

The pawls (216, 218) are moveable in an essentially axial direction responsive to pressure pulse of carrier liquid through the selected inlet pressure-activated valve (140). In a first (upper) position activated by maintaining a pulse of pressure by the carrier liquid **(****FIG. 12C**), the first pawl (216) engages with the first gear (262) to form the first ratchet. In the first (upper) position the second pawl (218) is released from the second gear (180) and hence from the second ratchet, allowing rotation of the lower disc (210) relative to the first body (210) to an extent allowed by the first ratchet **(FIGa. 12A** to **C**). In a second (lower) position **(FIGa. 12A** and **E**) in which the compliant region (214) is biased, the second pawl (218) engages with the second gear (180) to form the second ratchet. In the second (lower) position the first pawl (216) is released from the first gear (262) (**FIGs. D** and **E**) and hence from the first ratchet, allowing rotation of the lower disc (210) relative to the first body (100) to an extent allowed by the second ratchet. In **FIG. E**, the compliant region (214) has advanced one ratchet position from position b to c. The pressure-actuated double ratchet thus produces a partial rotation of the lower disc (210) at the start of the pulse (rise in pressure) and at the end of the pulse (drop in pressure), the combined rotation moving the pawls (216, 218) and hence the compliant region (214) a discrete revolute angle.

According to one aspect the selector unit (200) comprises a plurality of electro-mechanical actuators (e.g. piezo-electric actuator arms), each configured to apply a passive locking force to a clamping element of a corresponding inlet pressure-activated valves. Each electro-mechanical actuator may be normally locked. The plurality of electro-mechanical actuators may be controlled by a controller, configured to select a subset of inlet pressure-activated valves for un-locking responsive to a pressure pulse of the liquid carrier. The controller is preferably configured to activate one electro-mechanical actuators at a time, thereby unlocking one of the clamping elements at the time (150).

According to one aspect the clamping element (150) can slide freely (up and down) and may be slidably displaced responsive to a pressure pulse - similar to the situations shown in **FIGs. 4** and **5****.** When a clamping force is applied to one end of the clamping element (150), it pushes clamping element (150) against the diaphragm (144), thereby locking the inlet valve in a closed position as shown in **FIG. 13****.** The passive clamping force may be provided by a piezo-electric actuator arm (292). Each electro-mechanical actuators may be normally locked; with no electric current flowing, the arm (292) applies sufficient force to lock the clamping element (150) as shown, for instance, in **FIG. 13****.** When a current is applied through the piezo-electric arm it bends upward, releasing the force onto the clamping element (150) from the locked position as shown, for instance, in **FIG. 14****.**

According to another aspect the clamping element (150) is disposed with a compliant dome-shaped flange (152) biased to retain the clamping element (150) in an unlocked position as shown, for instance in **FIG. 15****.** This may be a position in which the valve is manufactured and shipped. When a clamping force is applied to one end of the flanged clamping element (150), it flattens the domed flange (152) and pushes clamping element (150) against the diaphragm (144), thereby locking the inlet valve in a closed position. Clamping force may be provided by a piezo-electric actuator arm (292). With no electric current flowing, the arm (292) applies sufficient force to lock the clamping element (150) as shown, for instance, in **FIG. 16****.** When a current is applied through the piezo-electric arm it bends upward, releasing the clamping element (150) from the locked position as shown, for instance, in **FIG. 17****.** The spring force of the domed flange lifts it away from the diaphragm.

The number of piezo-electric arms (292) may be the same as the quantity of inlet valves; in **FIG. 18****,** the piezo-electric arms (292, 292", 292"') radiate from a connected chassis (290). Each arm may have its own individual power connection so that each can opened or closed independent from each other. The arms by be controlled using an electronic controller which activates the piezo-electric arms one after the other. A power source may be provided to supply current to the piezo-electric elements and for the controller. The instruction can come wirelessly from the pump or by electrical connection via a special tubing.

The administration device (300) may be configured to administer the dose subcutaneously, intravenously, intramuscularly, orally, by inhalation, or by any other route. The administration device (300) connects to or contacts the subject and delivers the dose onto or into the subject. Where the dose is to be delivered subcutaneously, for example, the administration device may comprise a needle or cannula. It may be known in the art as a hypodermic infusion set. In the case of a wearable system, the administration device may comprise a housing for attachment to the skin of the subject such that the administration device can be worn for long period with the needle or cannula *in situ.* The first body may be dismountably attachable to the administration device such that it can be worn by the subject, the outlet port fluidly coupling with the needle or cannula. Alternatively, the first body (100) and administration device may be worn separately to avoid applying stress forces to the administration device by the relatively larger first body (100) and selector unit (200). The first body (100) and selector unit (200) may be fastened to the skin at a separate location with a separate adhesive sheet. A tubing length between the first body (100) and the administration device is typically short to avoid creation of a 'dead volume' requiring the use of addition liquid carrier with every new dose.

The cannula may be provided for intravenous administration, or administration into any cavity or vessel or space of the subject. The cannula may be a soft cannula - a flexible cannula. The soft cannula may be temporarily stiffened by a removable co-axial rigid cannula. The cannula may be a rigid cannula. Variations include administration device provided with a catheter. The administration device may be for administration of vaccines to different subjects; it is foreseen to be configured for removal of a needle after use and installation of an unused needle.

Shown in **FIG. 20** is an exemplary administration device (300) sometimes called hypodermal infusion set that is worn for prolonged periods by the subject and delivers one or more substances subcutaneously. It comprises an adhesive sheet (302) for attachment to the skin, a first coupling element (304) for attachment to a complementary second coupling element (104) on the first body (100), a cannula (306) for subcutaneous delivery of the substance in carrier liquid to the body, and an inlet connector (308) on first coupling element (304) connected to the cannula (306) for coupling to a complementary outlet connector (108) on the first body. The first coupling element (304), a cannula (306), inlet connector (308) are disposed on or within a second body (309) that is mounted on a non-adhesive side of the adhesive sheet (302). The first body (100) dismountably attaches to the administration device (300), as the respective coupling elements (104, 304) couple together. The second coupling element (104) is disposed with an outlet connector (108) that fluidly connects with the inlet connector (308) of the administration device (300). This allows the inlet connector (308) to be in fluid connection to the common outlet conduit (132). The outlet connector (108) is fluidly connected to the outlet port (136) via a conduit (106).

The administration device (300) may be configured to administer two different liquids via two different fluid conduits (308, 312) as shown, for instance, in **FIGs. 21A** to **D****.** The different liquids may contain different substances and/or carrier liquids (310, 314). A first fluid conduit (308) may be used to administer a first liquid (310) such as containing insulin and a second fluid conduit (312) to administer a second liquid (314) such as containing glucagon. Such dual liquid administration device (300) limits the need for separate devices (e.g. two infusion sets). According to the example of **FIGs. 21A** and **21B****,** the different fluid conduits (308, 312) connect outside (**FIGs. 21A**) or inside (**FIGs. 21B**) the second body (309), and are connected to a single lumen cannula (306). Thus, any mixing of the first (310) and second (314) liquids arises prior to passage through the single lumen of the cannula (306). According to the example of **FIGs. 21C** and **21D****,** the cannula (306) contains two lumens (a, b) each connect to a separate fluid conduit (308, 312). In **FIG. 21C****,** the lumens (a, b) are one within the other *e.g*. co-axially disposed. In **FIG. 21** **D,** the lumens (a, b) are one next to the other *i.e.* adjacent. Such double lumen cannulas have two outlets for the two different liquids in very close proximity to each other, thereby effecting mixing of the first (310) and second (314) liquids within the body. According to another example, a first cannula (306a) in fluid connection with the first fluid conduit (308), and a second cannula (306b) in fluid connection with the second fluid conduit (312), both first cannula (306a) and second cannula (306b) outlets arranged in close proximity to effect mixing of the first (310) and second (314) liquids within the body. Close proximity will be understood by the skilled and would depend on the bodily location of the cannulas; for most applications, it would be equal to or less than 5 cm apart. A dual liquid administration device (300) creates possibility for both liquids (310, 314) to react with each other in the body when delivered. For instance, a neutralising agent contained in a second liquid (314) might block the activity of a principal agent contained in a first liquid (310). Alternatively, an activating agent contained in a second liquid (314) might activate a principal agent contained in a first liquid (310).

A kit may be provided comprising the first body (100) and the selector unit (200). A kit may be provided comprising the administration device (400) and the selector unit (200). A kit may be provided comprising the first body (100), the administration device (400) and the selector unit (200).

The pump device typically contains a liquid reservoir and system for applying pressure. The pump device may be an electrically power pump (*e.g*. piezo-electric pump, piston pump, peristaltic pump, syringe pump). The pump device may be wearable. The pump device may be autonomous (*e.g*. battery operated). The pump device may be manually actuated, e.g. using a syringe. The pump may have similar characteristics to insulin pumps. Preferably the pump receives the data from a continuous glucose monitor so that the algorithm for a glucagon intervention can be automated. To facilitate the data integration (amount of delivered insulin) and convenience for the patient, the glucagon and insulin pump can be combined in one device.

The first body (100) described herein preferably holds at least 10 doses in a traditional powder form, each in a discrete storage reservoir. The outlet port (136) is connected to an administration device (*e.g*. subcutaneous infusion set) and the inlet port (138) connected to a pump. In a preferred aspect the first body (100) is clipped onto an infusion set (identical to those for subcutaneous insulin infusion) in such a way that the needle at the back of the first body (100) connects to the infusion set. The inlet port (138) of the first body (100) is connected to a pump by a connector for the pump tubing. The pump is similar to an insulin pump and can deliver liquid carrier on demand. When the pump is activated, liquid carrier is pumped into the common inlet channel.

The storage reservoir to be used is selected by unlocking the inlet valve to the reservoir. The selection of the inlet valve is done by the selector unit (200) that attaches to the first body (100). The flow of the liquid carrier mobilises the powdered substance and carries it further into the common outlet conduit (132). A region of the common outlet conduit (132) enhances the turbulence in the liquid flow. By doing so it stimulates a swirling action and therefore the mixing of the liquid carrier with the powdered substance. The resulting mixture flows out from the outlet port (136) into the infusion set and is deposited into the sub-cutis at the end of the infusion set cannula.

When not in use, the storage reservoirs are sealed to maintain the powder in a dry form. This is done by the containment of the channel in combination with the inlet and outlet pressure activated valves.

Interventions with glucagon open the possibilities to protect a diabetic patient from hypoglycemia. The intervention may be manually initiated by instructing the pump to deliver a dose. Preferably the pump is automatically activated when a risk is detected for low glucose. Automated glucagon delivery may receive input relating to one or more of the following:
- Continuous glucose monitoring (CGM) from continuous glucose sensor disposed on the subject. From this information the current glucose level is known and the rate of decline of the glucose level. These two parameters allow a prediction of when the glucose level is becoming too low to be safe. The CGM data is used by an algorithm to determine the timing of a glucagon intervention. Activation of the pump produces a pulse of pressure that actuates the present device and delivers a dose of glucagon.
- After delivery of a dose of glucagon and the combination with the evolution of the continuous glucose measurements, an algorithm may determine that a decline in the glucose level is still occurring or a raising of the glucose level is not sufficiently fast. In such case, another dose may be delivered.
- Knowledge of how much insulin has been given to the patient in the last few hours may be used to making better decisions on when to intervene and with how much glucagon. A patient with low glucose levels and a lot of insulin delivered in last few hours will see his glucose drop further. An earlier intervention with a dose of glucagon might be required.

The device (500) may be provided with the continuous glucose monitor (CGM). Superior protection is offered against low glucose levels when a problem is detected in advance. In such a combination, a pump may display CGM results after receiving the data from the CGM. In addition the pump may house a micro-processor storing an algorithm for determining glucagon dose delivery.

The device (500) may be provided with a dual pump. The ideal system combines the glucagon with the insulin pump in one set up. The pump may have two chambers: one for the carrier liquid and one for the insulin. Data on the amount of insulin delivered over the past few hours is better integrated in a glucagon delivery algorithm.

## Claims

1. Device (500) for storage and reconstitution of one or more discrete doses of a substance in a pressurised carrier liquid, comprising a first body (100) comprising:
- at least one discrete storage reservoir (110) configured for storage of the dose of substance, wherein the reservoir is in fluid connection with an inlet channel (112) and an outlet channel (114),
- wherein the inlet channel (112) is disposed with an inlet pressure-activated valve (140), configured to open responsive to a force of the pressurised carrier liquid,
- whereby the inlet channel (112) is connected at an inlet end to a common inlet conduit (130), and
- whereby the outlet channel (114) is connected at an outlet end to a common outlet conduit (132) configured for mixing the substance in the carrier liquid.

2. The device (500) according to claim 1, wherein the common outlet conduit (132) is provided with a region (134) dimensioned to facilitate turbulent flow over laminar flow, optionally having for a diameter D in metres, and a Reynolds number Re_{D} >4000.

3. The device (500) according to claim 1 or 2, wherein the outlet channel (114) is disposed with an outlet pressure-activated one-way valve (160), configured to open responsive to force of the pressurised liquid carrier exiting the storage reservoir (110).

4. The device (500) according to any of claims 1 to 3, wherein the outlet pressure-activated one-way valve (160) comprises:
- a diaphragm (164) provided in a wall (163) of the outlet channel (114), and
- a seating member (162) disposed in a void space of the outlet channel (114) wherein the diaphragm (164) forms a fluidly sealing contact with the seating member (164) to seal the outlet channel (114),
wherein the diaphragm (164) is provided across a recess (166) in the wall (163) of outlet channel (160) and is with an aperture (168) at an outlet side configured for the passage of back-flowing liquid into the recess (166) wherein back-flowing liquid fills the recess (166) via the aperture (168) expanding the diaphragm (164) and sealing it against the seating member (162) to prevent entry of the back-flowing liquid into the storage reservoir (110).

5. The device according to any of claims 1 to 4 having a plurality of reservoirs (110), wherein the reservoirs (110) and connected inlet (112) and outlet (114) channels are arranged in an arc or circle, the inlet channels (112) disposed towards a periphery, the outlet channels (114) arranged towards a centre, and the reservoirs (110) disposed between the inlet and outlet channels.

6. A device according to any of claims 1 to 5 where downstream of the inlet valve (140) a flow restrictor (400) is provided increasing the flow resistance (400) causing sufficient resistance to create sufficient pressure in the liquid flow to open the inlet valve (140)

7. The device (500) according to any of claims 1 to 6 wherein each of the inlet pressure-activated valves (140) is selectively lockable in a closed position.

8. The device (500) according to claim 7 wherein the lockable inlet pressure-activated valve (140) comprises:
- a diaphragm (144) provided in a wall (142) of the inlet channel (112),
- a seating member (146) disposed in a void space of the inlet channel (112), wherein the diaphragm (144) forms a fluidly sealing contact with the seating member (146) to seal the inlet channel (112),
- a moveable clamping element (150) configured to apply a locking force to the diaphragm (144) against the seating member (146) from an exterior of the wall (142) of the inlet channel (112) to lock it in a closed position.

9. The device (500) according to claim 8 further comprising a selector unit (200) configured for dismountable attachment to the first body (100), and to lock a selected subset of the inlet pressure activated-valves (140) in a closed position, thereby directing the pressurised carrier liquid through one or more inlet pressure-activated valves not in the subset.

10. The device (500) according to claim 9 wherein the selector unit (200) is configured to change the composition of the subset between consecutive pulses of the pressurised carrier liquid.

11. The device (500) according to claim 9 or 10, wherein the selector unit (200) comprises
- a plurality of electro-mechanical actuators (292) in a normally-locked state each configured to apply a locking force to a clamping element (150), and
- a controller configured to activate one electro-mechanical actuator at a time, thereby unlocking one of the clamping elements at the time (150).

12. The device (500) according to claim 9 or 10, wherein the selector unit (200) comprises a pressure-activated spring-loaded releasable ratchet mechanism configured to release a moveable pawl (216, 218) one ratchet position responsive to a pressure pulse of the carrier liquid, wherein the position of the pawl (216, 218) is in alignment relative to a position of the one or more clamping elements (150) in a non-locked position.

13. The device according to claim 12, wherein the selector unit (200) comprises
- an upper disc (260) and a lower disc (210) in mutual revolute relation around their respective central axes, the lower disc (210) provided for rotation relative to the first body (100), and the upper disc provided to be in fixed revolute relation with the first body (100),
- a spring (220) disposed between the lower disc (210) and the upper disc (260) to supply torque to rotate the lower disc (210) between ratchet positions,
- a first releasable ratchet comprising a first pawl (216) disposed on a compliant region (214) of the lower disc (210) and a first gear (262) disposed on the upper disc (260),
- a second releasable ratchet comprising a second pawl (21) disposed compliant region (214) of the lower disc (210) and a second gear (180) disposed on the first body (100),
- the first gear teeth (264', 264", 264"') and second gear teeth (182', 182", 182"') are in alignment relative to a positions of the clamping elements,
- wherein the lower disc (210) is configured to apply a locking force to a subset of clamping elements (150),
- wherein the compliant region (214) provided with the first pawl (216) and second pawl (218) is moveable between the first and second gears responsive to movement of the clamping element (150) under force of the pressurised carrier liquid, thereby releasing and moving the ratchet one ratchet position responsive to a pressure pulse.

14. A selector unit (200) as defined in any of claims 9 to 13.

15. A liquid administration device (300) for bodily subcutaneous administration of a first liquid (310) and a second liquid (314), comprising a first fluid conduit (308) for conveying the first liquid (310), and a second fluid conduit (312) for conveying the second liquid (314), the liquid administration device (300) further comprising:
- a cannula (306) having a single lumen in fluid connection with the first fluid conduit (308) and the second fluid conduit (312) arranged to effect mixing of the first (310) and second (314) liquids prior to conveyance through the cannula (306), or
- a single cannula (306) having a first lumen (306a) in fluid connection with the first fluid conduit (308), and a second lumen (306b) in fluid connection with the second fluid conduit (312), the first lumen (306a) outlet and second lumen (306b) outlet arranged to effect mixing of the first (310) and second (314) liquids within the body, optionally wherein the first cannula (306a) and a second cannula (306b) are disposed adjacently or coaxially,
or
- a first cannula (306a) in fluid connection with the first fluid conduit (308), and a second cannula (306b) in fluid connection with the second fluid conduit (312), both first cannula (306a) and second cannula (306b) outlets arranged in close proximity to effect mixing of the first (310) and second (314) liquids within the body,
optionally wherein the liquid administration device (300) is configured for dismountable attachment to the device (500) according to any of claims 1 to 13, and for fluid connection of the first (308) or second (312) fluid conduit to the common outlet conduit (132).
